# EUROPEAN PATENT SPECIFICATION

(11) **EP 1 229 871 B1**
(45) Date of publication and mention of the grant of the patent: **02.02.2005**
(21) Application number: 00976509.0
(22) Date of filing: 07.11.2000
(51) Int. Cl.: A61F 2/28, A61C 8/00

(54) **AN ELEMENT INTENDED TO BE IMPLANTED IN BODY TISSUE, AND A DEVICE FOR MANUFACTURING SUCH AN ELEMENT**
ELEMENT ZUR IMPLANTATION IN KÖRPERGEWEBE UND GERÄT ZU DESSEN HERSTELLUNG
ELEMENT DESTINE A ETRE IMPLANTE DANS UN TISSU CORPOREL ET DISPOSITIF PERMETTANT DE FABRIQUER CET ELEMENT

(30) Priority: 17.11.1999 SE 9904145
(43) Date of publication of application: 14.08.2002
(73) Proprietor: Mediteam Dental AB, 433 63 Sävedalen (SE)
(72) Inventor: Wennerberg, Ann, S-431 39 Mölndal (SE); Rosén, Bengt, Göran, S-302 41, Halmstad (SE)
(74) Representative: Cederbom, Hans Erik August
(86) International application number: PCT/SE2000/002171
(87) International publication number: WO 2001/035871

(56) References cited:
- EP-A1- 0 388 576
- WO-A1-95/09583
- WO-A1-95/13102

## Description

The present invention relates to an element intended to be implanted in body tissue, especially bone tissue, which exhibits a controlled surface structure on at least that part of the implant which is so arranged as to be in contact with bone.

Previously disclosed is the fact that the surface topography of an implant surface is of significance to how well it is integrated with surrounding tissues. For example, Wennerberg at al have described how the surface topography influences the healing process for implants in bone; ref. Wennerberg, PhD Thesis 1996, and Wennerberg, 1998 "A histomorphometric evaluation of screw-shaped implants each prepared with two surface roughnesses".

All implants available on the market exhibit more or less stochastic surface structures, which are the result of, for example, turning processes, milling processes, etching or blasting processes. These processes, among others, or combinations of them are used to control the topography of the surfaces. For example, Patent Application SE 7902035-0 describes an implant surface which is characterized by the fact that at least the surface layer consists of a material containing titanium, and that the surface is micro-pitted in the range from 10-1000 nm, and preferably 10-300 nm. The results are good for the surface, which is explained by the fact that the pits are of the same order of magnitude as the cells in the surrounding tissue.

Research shows that it is desirable to be able to control the surface structure better than is the case in conjunction with the use of the aforementioned traditional methods for producing surfaces with different surface roughness. It is highly likely that surfaces with an even more controlled structure may be desirable in order further to improve and accelerate the integration of an implant in the surrounding tissue. Research is being conducted into, among other things, surfaces with a higher degree of control. These are generally referred to as micro-fabricated surfaces. Tools commonly used in the production of these surfaces include laser machining, or so-called laser ablation, whereby the surface layer is vaporized in a specific pattern either by positioning the laser and causing pits to be burnt at given distances, or by causing various optical arrangements to burn a particular pattern into the surface.

Other previously disclosed methods include the so-called lithographic methods (photo lithography and electro lithography) and special variants of these, for example so-called stamping, by means of which a pattern can be obtained with more control over the detail. Details which can be controlled are the distribution of patterns over surfaces and the depth of the pattern. These methods very often use some form of etching technology (wet, dry or electrochemical).

The advantage of the lithographic methods is that production can be made rather more rational than with the methods based on laser machining. When using laser methods, the laser beam must be repositioned between each stage of the machining process, whereas the lithographic methods can produce patterns over larger components at each machining stage.

A surface which is the result of laser machining is described in patent publication WO 95-09583. The pits in this case exhibit a mean diameter of 100-200 µm and can be likened to craters, since they are surrounded by raised areas which extend up above the surrounding implant surface and in this way form a border which fully or partially surrounds the pore.

One example shows that the pores have an average diameter of 150 µm and a depth of a similar order of magnitude. The crater contours are shown here to be raised by about 5 µm above the surrounding surface, and the average distance between the pores is about 120 µm. Other previously disclosed methods for creating the desired surface roughness are blasting, etching or plasma spraying. Blasting is described in, for example, patent publications WO 92/05745 and WO 95/13102.

Patent EP 0 388 576 shows an implant surface produced by a combination of blasting and etching. The reason for the combination is to create a macrostructure with pores of the order of 10 µm in size with a superimposed microstructure with a pore size of 2 µm. It is stated in the patent that the surface exhibits improved results in animal experiments.

An example of plasma-sprayed surfaces can be found in EP 0 222 853. The surface, which is the result of a plasma process, is a coarse and sponge-like structure.

Reference is also made to US-A-4,865,603, in which it is stated that "micro-texture is created by sequentially forming two sets of recesses in the outer surface of the prosthesis" .In conjunction with this, it is stated that the purpose is to achieve a "readily controlled process" in order to create the surface structure. Methods mentioned are "machine tooling, configurated coasting, laser metal removal and electrochemical", etc. This patent describes changes in the surface structure of the order of 300-700 µm, and the intention of these surfaces is to create mechanical locking to the bone at macroscopic level, unlike the intention in the other patents referred to, where the surface structure is controlled on a significantly smaller scale with a view to accelerating the healing process and the formation of new bone. The intention is also for the implant to be used to anchor joint prostheses, in the main hip prostheses. The production methods that are used (blasting, etching and plasma spraying) are characterized by the fact that the resulting surface has an irregular topography which in itself exhibits a surface roughness which is repeatable if it is quantified by static surface roughness measurement methods (Rc, Sc values, etc.). On the other hand, the surfaces are not well controlled in such a way that the pattern can be clearly described at the level of micrometres and below, which is a precondition for optimizing the effect of the implant on the surrounding tissue. The sequence which leads to anchorage is determined by the interaction between the tissues and the surface of the implant during the healing phase. Mechanisms which control this interaction take place at molecular/protein/cell and tissue level. In order to be able to study the effect of the surface topography on the healing process more specifically, it is necessary to be able to control the structure of the surface in more detail. Attempts at this are described above with reference to laser machining and photo lithography. These methods are excellent if applied in a research context, although it is very difficult and expensive to reproduce the surfaces in large-scale production. The greater the control it is wished to have over the surface, the more complicated the production process becomes.

The principal object of the present invention is thus, in the first instance, to solve the aforementioned problems by simple means by the manufacture in a repeatable fashion of elements of the indicated kind, which exhibit surfaces with control at sub-micro level.

The aforementioned object is achieved by means of an element in accordance with the present invention, which is characterized essentially in that the surface and the form of that part of the implant that is so arranged as to be in contact with the bone are produced in a common operation, so that the surface exhibits controlled isotropic surface topography with high repeatability.

The invention also relates to a method for the production of elements of the kind referred to above for the purpose of enabling the production in a repeatable fashion of elements of a kind which exhibit surfaces with control at sub-micro level to facilitate scaling-up the method to an industrial level and to increase the repeatability in relation to previously disclosed earlier methods which included laser ablation or lithographic methods, and now in a common operation.

A further object of the invention is thus to find a method which solves the aforementioned problems effectively.

The aforementioned further object is achieved by means of a method in accordance with the embodiment described above, which is characterized essentially in that both the surface and the form of that part of the implant that is arranged to be in contact with bone are produced in a common forming operation, so that the surface is given a controlled isotropic surface topography and with high repeatability.

Arrangements for the manufacture of implant elements of the kind intended in accordance with the invention have not made it possible to produce both the macrostructure and the microstructure of the element in a common production operation together with a controlled isotropic surface topography on the surface part of the element.

A final object of the invention is thus to provide means which satisfy the aforementioned wishes.

The aforementioned final object is achieved by means of an arrangement in accordance with the present invention, which is characterized essentially in that a tool for the manufacture of the elements in question exhibits a forming surface that is the reverse of the intended structure of the element that it is intended to form, in respect of both its macrostructure and its microstructure.

The invention is described below as a number of preferred illustrative embodiments, in conjunction with which reference is made to the drawings, in which:

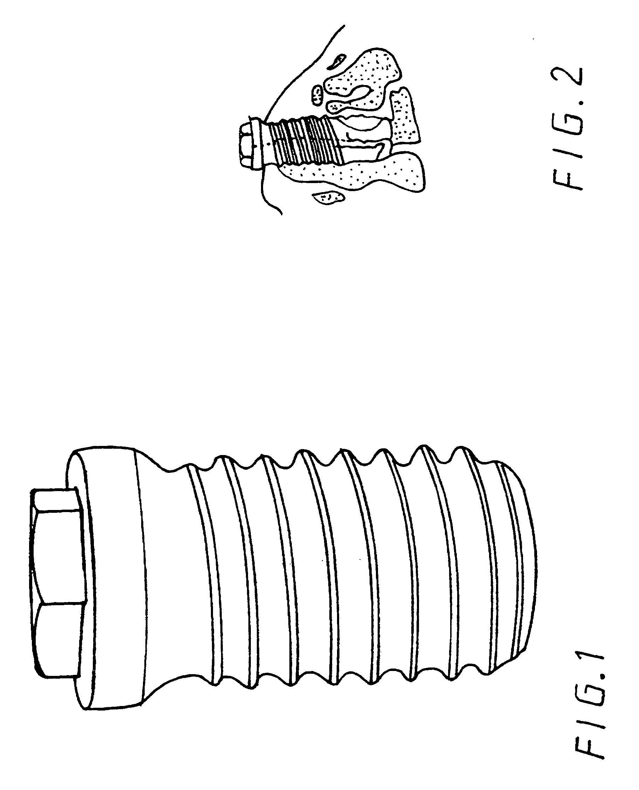

Fig. 1 shows an illustrative embodiment of an implant element which is provided with external threads;
Fig. 2 shows an example of an implant fixed in bone;

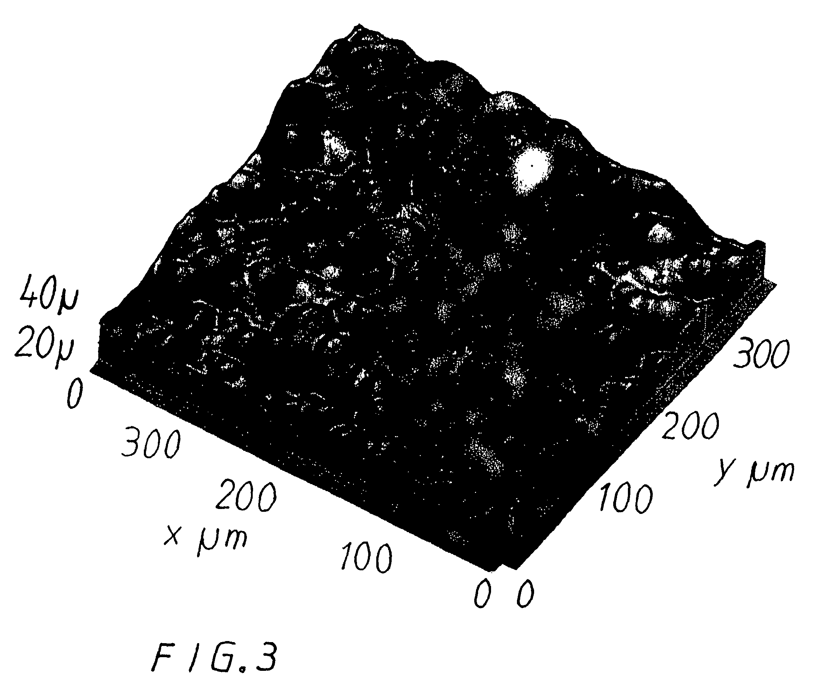

Fig. 3 shows the topography of a first example of a tool for the manufacture of elements and with a coarser surface;

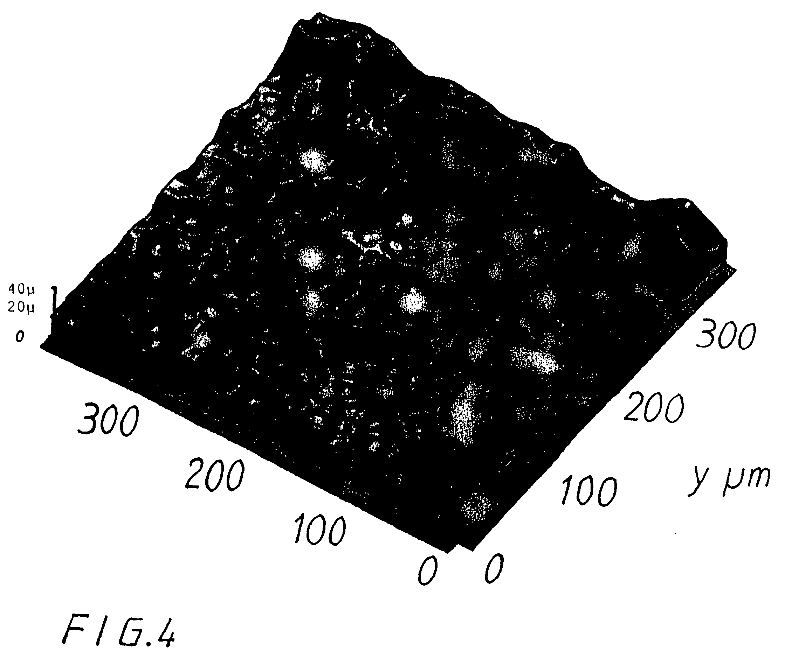

Fig. 4 shows the topography of an element processed with a tool in accordance with the foregoing;

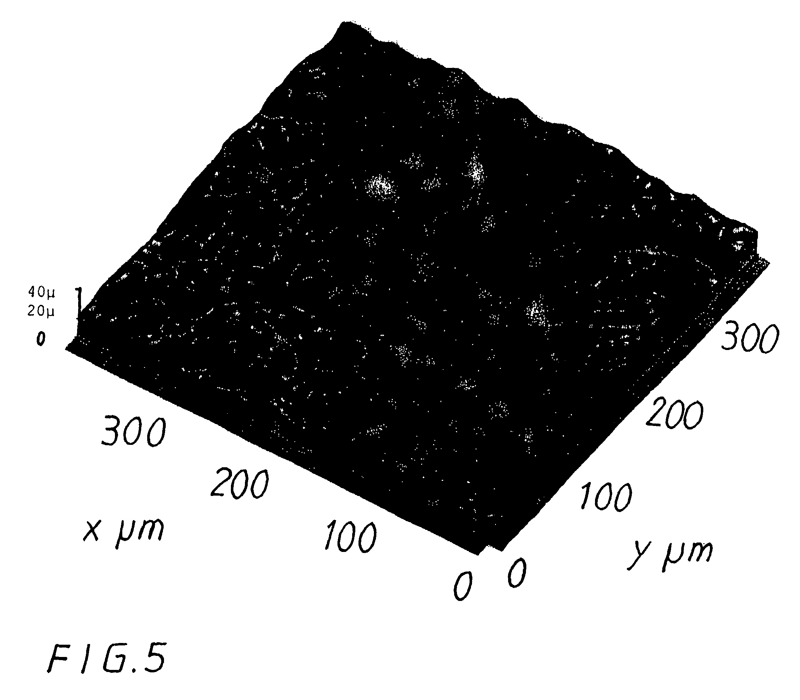

Fig. 5 shows the topography of a second example of a tool for the manufacture of elements and with a finer surface;

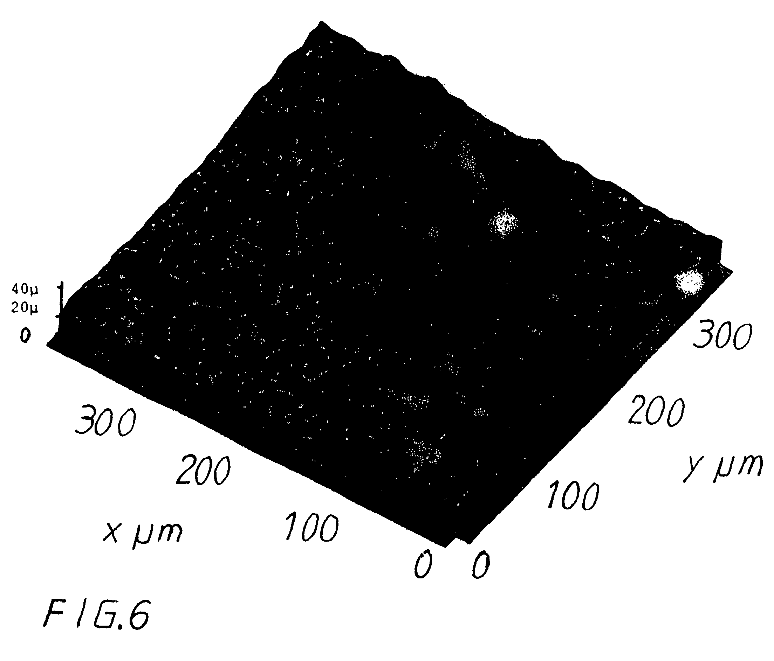

Fig. 6 shows the topography of an element processed with a tool in accordance with the second example.

The invention is described below as an example of implant elements 1 provided with threads 2 which are present for the purpose of increasing the fixing capability of the elements 1.

The invention can also be applied, however, to elements which lack threads, for example smooth, uniformly thick or conical fixing pins, etc.

Nevertheless, the following has emerged in respect of biological interest:

Increasing the surface roughness of screw-shaped implants from that obtained in conjunction with the turning of titanium has been found both to be associated with more rapid healing and to provide more bone in contact with the implant. This offers the possibility of shorter healing times, and thus shorter waiting times for the patients. Turned implants have a distinct orientation of their surface structure (anisotropic), whereas blasted implants, for example, are isotropic. This characteristic can also provide an explanation for the increased amount of bone contact that is often observed in conjunction with implants with an increased surface area. It is important to provide the surface of an implant with isotropic surface roughness in the area identified as optimal in previous studies, in such a way as to provide very high repeatability. Small changes can bring about a difference in the tissue response. That is why repeatability is significant from the point of view of the prognosis.

Moreover, the manufacture of screws with the help of rolling has already been disclosed for some time, although providing the surface with a controlled isotropic topography is novel. This method means that the thread profile of the screw and its surface topography are manufactured in a common operation. The surface topography of the tool results in a negative image on every unit manufactured. The method gives very high repeatability from batch to batch. An example of a processing method used to obtain the macrostructure of the implant is thread-rolling or a similar method in which the thread profile is produced with plastic deformation and reproduction of the screw material in contact with the thread-rolling tool. The topography of the thread-rolling tool that is reproduced in the surface of the screw can be manufactured by coating with the TopoCrom method, for example, Patent No. DE 334 122 C2, in which spherical peaks are reproduced as cavities in the surface of the screw, or by the use of equivalent technology, for example etching, in which formed pits in the surface of the tool are reproduced as peaks in the surface of the screw, or by some other coating or machining process.

The screws that are manufactured are physically made with a standard thread with a pitch of 0.6 mm. The microtopography is characterized by pits or peaks with peaks and/or valleys with a height and/or a depth of 3-60 µm and a density of micropits and/or micropeaks of 50-5000/mm².

The invention is described more specifically as comprising an element 1, which consists of a titanium material or some other appropriately bone-compatible material with which to heal and fuse together, and which is intended to be implanted in body tissue, in particular bone tissue 3, such as the human jawbone in the case of a tooth implant or an implant for other odontological operations, which exhibits a controlled surface structure 4, 4¹ on at least that part of the implant 1 which is so arranged as to be in contact with bone. In accordance with the invention, both the surface 5, 5¹ and the form 9, 9¹ of the part 4A of the implant 1 which is so arranged as to be in contact with bone are manufactured in a common operation, so that the surface 5, 5¹ exhibits controlled isotropic surface topography. This provides high repeatability.

As stated above, it is preferably threads 2 which exhibit the aforementioned controlled isotropic surface topography 4, 4¹.

The surface 5, 5¹ of the aforementioned threads 2 or other parts of the implant 1 exhibits pits 6, 6¹ and/or peaks 7, 7¹, with peaks and/or valleys with a height and/or a depth of 3-60 µm and a density of micropits and/or micropeaks of 50-5000/mm².

A method for the production of such implant elements 1 proceeds in such a way that both the surface 5, 5¹ and the form 9, 9¹ of the part 4A of the implant 1 which is so arranged as to be in contact with bone are manufactured in a common forming operation, so that the surface 5, 5¹ of the manufactured implant 1 is given a controlled isotropic surface topography 4, 4¹ and with high repeatability.

In those cases in which the implant element 1 exhibits threads 2 or other suitable fixing devices and is executed as an implant screw, preferably made of a homogeneous material or with a coating of a titanium material or some other bone-compatible material, the screw is manufactured so that both its macrostructure, i.e. its threads 2, and its microstructure, i.e. its specific and desired surface structure, are produced in a common production operation and with a controlled isotropic surface topography in the threads 2, etc.

The thread profile of the screw is appropriately manufactured by thread rolling or by means of other compression of the material in the screw 1 so that the thread profile is formed by plastic deformation and reproduction of the surface form 8, 8¹ of the thread-rolling tool or some other forming tool 10, 10¹.

The topography of the screw 1 is thus achieved by reproducing an uneven but defined surface 8, 8¹ on the thread-rolling tool 10, 10¹ as the reverse of the form of the desired surface 5, 5¹, i.e. pits 11, 11¹ in the tool 10, 10¹ become peaks 7, 7¹ on the implant 1, and peaks 12, 12¹ in the tool 10, 10¹ become pits 6, 6¹ on the implant 1.

The problem of producing elements of the indicated kind in a repeatable fashion, which exhibit surfaces with control at the sub-micro level by a method that is readily capable of being scaled up to an industrial level is thus solved through the present invention. The repeatability also increases in relation to the methods, which consist of laser ablation or lithographic methods.

Production thus takes place in a single stage with regard to both the macrostructure, e.g. threads, and the microstructure, the structure of the surface 5, 5¹ at µm level.

The embodiment derives from the fact that both the macrostructure and the microstructure are created by pressing the surface to produce the desired structure. If the macrostructure is a thread, the production method can be likened to thread-rolling, which is a production method commonly used in the production of machine screws; for example, the roller jaws in this case can be provided, in addition to the form for the thread profile, with a surface structure at µm level. This structure produces an impression in the surface in a highly repeatable fashion. Titanium, which is a familiar and well-documented material in implants, is an ideal material for production in this way.

One illustrative embodiment involves the use of thread-rolling, for which the roller jaws have been provided with a surface layer containing the desired surface structure. One way of producing the surface structure is, for example, the commercially available, so-called Toppocrom method, in accordance with the above patent. This is marketed by WMV Angelagen AG, Germany, and is used in particular to create a µ structure on rollers for sheet metalworking. The intention in many cases is to cause lubricant to bond to the surfaces. In addition to better repeatability, this method also gives a surface which is not chemically contaminated by chemicals that are used in the lithographic processes, for example, or by the fact that chemical reactions are triggered by the heating that is unavoidable in conjunction with laser machining.

It is thus possible to utilize an applied surface coating on the thread-rolling tool in order, when it is actuated, to obtain the desired impression on the implant 1.

An arrangement of the kind intended in accordance with the invention, which lends itself to use for the production of elements 1 intended for implantation in body tissue, in particular bone tissue 3, and which exhibits a controlled surface structure on at least that part of the implant that is so arranged as to be in contact with bone, comprises a tool which is arranged for the manufacture of an element of the kind in question. The aforementioned tool 10, 10¹ exhibits a forming surface which is the reverse of the intended structure, which is so arranged as to be formed on the element 1 that it is intended to form, in respect of both its macrostructure and its microstructure.

As stated above, the tool may preferably be in the form of a press tool such as roller jaws, where the macrostructure of the element includes threads 2.

The surface of the aforementioned tool 10, 10¹ exhibits an applied surface structure at µm level for the purpose of forming a controlled isotropic surface topography in the element 1 with high repeatability.

The invention thus provides for the simple and yet precise manufacture of implant elements 1, which exhibit the desired surface structure on all surfaces without problem. The invention is not, however, restricted to what is shown in the drawings and described above, but may be varied within the scope of the Patent Claims without departing from the idea of invention.

## Claims

1. Element (1) intended to be implanted in body tissue, especially bone tissue (3), which exhibits a controlled surface structure (4, 4¹) on at least that part of the implant (1) which is so arranged as to be in contact with bone, ***characterized in that*** the surface (4, 4¹) and the form of that part of the implant (1) that is so arranged as to be in contact with bone are produced in a common operation, so that the surface (4, 4¹) exhibits controlled isotropic surface topography, which gives high repeatability.

2. Element in accordance with Patent Claim 1, ***characterized in that*** it is threads (2) which exhibit the aforementioned controlled isotropic surface topography (4, 4¹).

3. Element in accordance with Patent Claim 2, ***characterized in that*** the surface (5, 5¹) of the aforementioned element (1) exhibits pits (6, 6¹) and/or peaks (7, 7¹), with peaks and/or valleys with a height and/or a depth of 3-60 µm and a density of micropits and/or micropeaks of 50-5000/mm².

4. Method for the production of an element (1) intended to be implanted in body tissue, especially bone tissue (3), which exhibits a controlled surface structure (4, 4¹) on at least that part of the implant (1) which is so arranged as to be in contact with bone in accordance with one or other of Patent Claims 1-3, ***characterized in that*** both the surface (5, 5¹) and the form (9, 9¹) of the part (4A) of the implant (1) which is so arranged as to be in contact with bone are manufactured in a common forming operation, so that the surface (5, 5¹) exhibits a controlled isotropic surface topography (4, 4¹) and with high repeatability.

5. Method in accordance with Patent Claim 4, in conjunction with which an element (1), which exhibits threads (2) or other fixing devices, is executed as an implant screw (1) preferably made of titanium material or some other bone-compatible material, **characterized in that** the screw (1) is manufactured so that both its macrostructure, i.e. its threads (2), and its microstructure, i.e. its surface structure, are produced in a common production operation and with a controlled isotropic surface topography in the threads (2).

6. Method in accordance with Patent Claim 5, ***characterized in that*** the thread profile of the screw is appropriately manufactured by thread rolling or by means of other compression of the material in the screw (1) so that the thread profile is formed by plastic deformation and reproduction of the surface form (8, 8¹) of the thread-rolling tool (10, 10¹).

7. Method in accordance with Patent Claim 6, ***characterized in that*** the topography of the screw (1) is achieved by reproducing an uneven but defined surface (8, 8¹) on the thread-rolling tool as the reverse impression, i.e. pits (11, 11¹) in the tool (10, 10¹) become peaks (7, 7¹) on the implant (1), and peaks (12, 12¹) in the tool (10, 10¹) become pits (6, 6¹) on the implant (1).

8. Method in accordance with Patent Claim 7, ***characterized in that*** an applied surface coating on the thread-rolling tool is used to obtain the reversed impression on the implant.

9. Arrangement for the manufacture of an element intended to be implanted in body tissue, especially bone tissue (3), which exhibits a controlled surface structure on at least that part (4A) of the implant (1) which is so arranged as to be in contact with bone in accordance with one or other of Patent Claims 1-3, ***characterized in that*** a tool (10, 10¹) for the manufacture of the element (1) in question exhibits a forming surface (8, 8¹) that is the reverse of the intended structure (4, 4¹) of the element (1) that it is intended to form, in respect of both its macrostructure and its microstructure.

10. Arrangement in accordance with Patent Claim 9, ***characterized in that*** the tool is in the form of a press tool, such as roller jaws, where the macrostructure of the element includes threads (2).

11. Arrangement in accordance with Patent Claim 10, ***characterized in that*** the surface of the aforementioned tool (10, 10¹) exhibits an applied surface structure at µm level for the purpose of forming a controlled isotropic surface topography on the element (1) with high repeatability.

## Patentansprüche

1. Element (1) zum Implantieren in Körpergewebe, insbesondere Knochengewebe (3), das eine gesteuerte Oberflächenstruktur (4, 4¹) an zumindest demjenigen Teil des Implantats (1) aufweist, der in Kontakt mit dem Knochen angeordnet ist,
**dadurch gekennzeichnet,**
**dass** die Oberfläche (4, 4¹) und die Form dieses Teils des Implantats (1), das in Kontakt mit dem Knochen angeordnet ist, in einem gemeinsamen Betriebsablauf hergestellt werden, so dass die Oberfläche (4, 4¹) eine gesteuerte isotrope Oberflächentopographie aufweist, die eine hohe Wiederholbarkeit zur Folge hat.

2. Element nach Anspruch 1,
**dadurch gekennzeichnet,**
**dass** es Gewindegänge (2) umfasst, die die vorher erwähnte gesteuerte isotrope Oberflächentopographie (4, 4¹) aufweisen.

3. Element nach Anspruch 2,
**dadurch gekennzeichnet,**
**dass** die Oberfläche (5, 5¹) des vorher erwähnten Elements (1) Vertiefungen (6, 6¹) und/oder Spitzen (7, 7¹) aufweist, wobei die Spitzen und/oder Täler eine Höhe und/oder eine Tiefe von 3 bis 60 µm aufweisen und eine Dichte von Mikrovertiefungen und/oder Mikrospitzen im Bereich von 50 bis 5000/mm² liegt.

4. Verfahren zur Herstellung eines Elementes (1) zum Implantieren in Körpergewebe, insbesondere Knochengewebe (3), das eine gesteuerte Oberflächenstruktur (4, 4¹) an zumindest demjenigen Teil des Implantats (1) aufweist, das in Kontakt mit dem Knochen angeordnet ist, nach einem der Ansprüche 1 bis 3,
**dadurch gekennzeichnet,**
**dass** sowohl die Oberfläche (5, 5¹) als auch die Form (9, 9¹) des Teils (4A) des Implantats (1), das in Kontakt mit dem Knochen angeordnet wird, in einem gemeinsamen Formbetriebsablauf hergestellt werden, so dass die Oberfläche (5, 5¹) eine gesteuerte isotrope Oberflächentopographie (4, 4¹) und eine hohe Wiederholbarkeit aufweist.

5. Verfahren nach Anspruch 4,
bei dem ein Element (1), das Gewindegänge (2) oder andere Befestigungseinrichtungen aufweist, als eine Implantatschraube (1) ausgebildet wird, die bevorzugt aus Titanmaterial oder einem anderen knochenkompatiblen Material hergestellt wird,
**dadurch gekennzeichnet,**
**dass** die Schraube (1) so hergestellt wird, dass sowohl ihre Makrostruktur, d.h. ihre Gewindegänge (2), als auch ihre Mikrostruktur, d.h. ihre Oberflächenstruktur, in einem gemeinsam Produktionsbetriebsablauf und mit einer gesteuerten isotropen Oberflächentopographie in den Gewindegängen (2) hergestellt werden

6. Verfahren nach Anspruch 5,
**dadurch gekennzeichnet,**
**dass** das Gewindeprofil der Schraube geeignet durch Gewindewalzen oder durch eine andere Kompression des Materials in der Schraube (1) hergestellt wird, so dass das Gewindeprofil durch plastische Verformung und Reproduktion der Oberflächenform (8, 8¹) des Gewindewalzwerkzeugs (10, 10¹) ausgebildet wird.

7. Verfahren nach Anspruch 6,
**dadurch gekennzeichnet,**
**dass** die Topographie der Schraube (1) dadurch erreicht wird, dass eine unebene, aber definierte Oberfläche (8, 8¹) an dem Gewindewalzwerkzeug als umgekehrter Abdruck bzw. umgekehrte Prägung reproduziert wird, d.h. Vertiefungen (11, 11¹) in dem Werkzeug (10, 10¹) werden Spitzen (7, 7¹) an dem Implantat (1), und Spitzen (12, 12¹) in dem Werkzeug (10, 10¹) werden Vertiefungen (6, 6¹) an dem Implantat (1).

8. Verfahren nach Anspruch 7,
**dadurch gekennzeichnet,**
**dass** eine aufgebrachte Oberflächenbeschichtung an dem Gewindewalzwerkzeug dazu verwendet wird, den umgekehrten Abdruck an dem Implantat zu erhalten.

9. Anordnung zur Herstellung eines Elementes, das zum Implantieren in Körpergewebe, insbesondere Knochengewebe (3) bestimmt ist und eine gesteuerte Oberflächenstruktur an zumindest demjenigen Teil (4A) des Implantats (1) aufweist, das in Kontakt mit dem Knochen angeordnet ist, nach einem der Ansprüche 1 bis 3,
dadurch gekennzeichne t,
dass ein Werkzeug (10, 10¹) für die Herstellung des betreffenden Elementes (1) eine Formoberfläche (8, 8¹) aufweist, die sowohl bezüglich ihrer Makrostruktur als auch ihrer Mikrostruktur der umgekehrten beabsichtigten Struktur (4, 4¹) des Elementes (1), die geformt werden soll, entspricht.

10. Anordnung nach Anspruch 9,
**dadurch gekennzeichnet,**
**dass** das Werkzeug in der Form eines Presswerkzeuges vorgesehen ist, wie beispielsweise Klemmwalzen, wobei die Makrostruktur des Elementes Gewindegänge (2) umfasst.

11. Anordnung nach Anspruch 10,
**dadurch gekennzeichnet,**
**dass** die Oberfläche des vorher erwähnten Werkzeuges (10, 10¹) eine aufgebrachte Oberflächenstruktur auf einem µm-Niveau aufweist, um eine gesteuerte isotrope Oberflächentopographie an dem Element (1) mit hoher Wiederholbarkeit auszubilden.

## Revendications

1. Elément (1) destiné à être implanté dans un tissu corporel, plus particulièrement dans un tissu osseux (3), qui présente une structure de surface contrôlée (4, 4¹) sur au moins une partie de l'implant (1) prévue pour être en contact avec l'os, **caractérisé en ce que** la surface (4, 4¹) et la forme de cette partie de l'implant (1) prévue pour d'être en contact avec l'os sont issues d'une même opération, de sorte que la surface (4, 4¹) présente une topographie de surface isotrope contrôlée, qui procure une reproductibilité élevée.

2. Elément selon la revendication 1, **caractérisé en ce que** les filetages (2) présentent la topographie de surface isotrope contrôlée (4, 4¹) susmentionnée.

3. Elément selon la revendication 2, **caractérisé en ce que** la surface (5, 5¹) de l'élément susmentionné (1) comporte des creux (6, 6¹) et/ou des pointes (7, 7¹), ces creux et/ou ces pointes présentant une hauteur et/ou une profondeur de 3 à 60 µm et une densité de micro-creux et/ou de micro-pointes de 50 à 5000/mm².

4. Procédé de fabrication d'un élément (1) destiné à être implanté dans un tissu corporel, plus particulièrement dans un tissu osseux (3), qui présente une structure de surface contrôlée (4, 4¹) sur au moins une partie de l'implant (1) prévue pour être en contact avec l'os selon l'une quelconque des revendications 1 à 3, **caractérisé en ce qu'**à la fois la surface (5, 5¹) et la forme (9, 9¹) de la partie (4A) de l'implant (1) prévue pour être en contact avec l'os sont issues d'une même opération de façonnage, de sorte que la surface (5, 5¹) présente une topographie de surface isotrope contrôlée (4, 4¹) et de reproductibilité élevée.

5. Procédé selon la revendication 4, et en combinaison avec celle-ci dans laquelle un élément (1), qui présente des filetages (2) ou d'autres dispositifs de fixation, est réalisé en tant que vis d'implant (1) préférentiellement fabriqué en titane ou en d'autres matières compatibles avec l'os, **caractérisé en ce que** la vis (1) est fabriquée de sorte qu'à la fois sa macrostructure, c'est-à-dire ses filetages (2), et sa microstructure, c'est-à-dire sa structure de surface, sont issues d'une même opération de fabrication et avec une topographie de surface isotrope contrôlée pour les filetages (2).

6. Procédé selon la revendication 5, **caractérisé en ce que** le profil de filetage de la vis est fabriqué de manière appropriée par roulage des filets ou au moyen d'une autre compression de la matière de la vis (1) afin que le profil de filetage soit formé par déformation plastique et reproduction de la forme de surface (8, 8¹) de l'outil de laminage des filets (10, 10¹).

7. Procédé selon la revendication 6, **caractérisé en ce que** la topographie de la vis (1) est obtenue par reproduction d'une surface inégale mais définie (8, 8¹) sur l'outil de roulage des filets en impression inverse, c'est-à-dire que les creux (11, 11¹) dans l'outil (10, 10¹) deviennent des pointes (7, 7¹) sur l'implant, et que les pointes (12, 12¹) dans l'outil (10, 10¹) deviennent des creux (6, 6¹) sur l'implant.

8. Procédé selon la revendication 7, **caractérisé en ce qu'**un revêtement de surface appliqué sur l'outil de roulage des filets est utilisé pour obtenir l'impression inversée sur l'implant.

9. Dispositif de fabrication d'un élément destiné à être implanté dans un tissu corporel, plus particulièrement dans un tissu osseux (3), qui présente une structure de surface contrôlée sur au moins une partie (4A) de l'implant prévue pour être en contact avec l'os selon l'une quelconque des revendications 1 à 3, **caractérisé en ce qu'**un outil (10, 10¹) pour la fabrication dudit élément (1) présente une surface de conformation (8, 8¹) inverse à celle prévue pour la surface (4, 4¹) de l'élément (1) destiné à être formé, en ce qui concerne sa macrostructure et de sa microstructure.

10. Dispositif selon la revendication 9, **caractérisé en ce que** l'outil est sous la forme d'un outil de presse, tel que des mâchoires à rouleaux, où la macrostructure de l'élément comporte des filetages (2).

11. Dispositif selon la revendication 10, **caractérisé en ce que** la surface de l'outil susmentionné (10, 10¹) présente une structure de surface appliquée au niveau micrométrique dans le but de former une topographie de surface isotrope contrôlée sur l'élément (1) de reproductibilité élevée.
